# EUROPÄISCHE PATENTSCHRIFT

(11) **EP 2 573 062 B1**
(45) Veröffentlichungstag und Bekanntmachung des Hinweises auf die Patenterteilung: **04.04.2018**
(21) Anmeldenummer: 12181518.7
(22) Anmeldetag: 23.08.2012
(51) Int. Cl.: C07C 45/40, C07C 51/34, C07C 67/333, C07C 69/738, C12P 7/64, C07C 47/02, C12P 7/24

(54) **Ozonolyse von ungesättigten Fettsäuren und Derivaten davon**
Ozonolysis of unsaturated fatty acids and derivatives of same
Ozonolyse d'acides gras insaturés et de leurs dérivés

(30) Priorität: 23.09.2011 DE 102011083285
(43) Veröffentlichungstag der Anmeldung: 27.03.2013
(73) Patentinhaber: Evonik Degussa GmbH, 45128 Essen (DE)
(72) Erfinder: Ullrich, Matthias, 34132 Kassel (DE); Hannen, Peter, 45701 Herten (DE); Roos, Martin, 45721 Haltern am See (DE)

(56) Entgegenhaltungen:
- PRYOR, DAS AND CHURCH: "Then Ozonisation of Unsaturated Fatty Acids", CHEM. RES. TOXICOL., Bd. 4, 1991, Seiten 341-348, XP002690849,

## Beschreibung

Die Erfindung betrifft ein Verfahren zur Ozonolyse von ungesättigten Fettsäuren und Derivaten davon umfassend die Schritte a) Ozonolyse einer ungesättigten Fettsäure oder eines Derivates davon in einer organischen Phase umfassend ein organisches Lösungsmittel, und b) Kontaktieren der organischen Phase mit einer wässrigen Phase umfassend Katalase und bevorzugt einen Puffer, wobei die ungesättigte Fettsäure oder das Derivat davon eine lineare Kette mit wenigstens acht Kohlenstoffatomen aufweist, sowie die Verwendung von Katalase zur Entfernung reaktiver Sauerstoffspezies aus einem Reaktionsgemisch umfassend Ozonolyseprodukte einer ungesättigten Fettsäure oder eines Derivates davon.

Die Ozonolyse ist ein in der synthetischen organischen Chemie etabliertes Verfahren zur Spaltung einer C=C-Doppelbindung eines ungesättigten Kohlenwasserstoffes unter Bildung zweier Carbonylverbindungen. Bei letzteren kann es sich insbesondere um Carbonsäure, Aldehyde und Alkohole handeln. Mechanistisch umfasst die Ozonolyse eine [3+2]-Cycloaddition des Ozons an die Doppelbindung, die ein Primär-Ozonid liefert, ein instabiles Zwischenprodukt, das zu einem Aldehyd und einem Carbonyloxid zerfällt. Letzteres kann entweder polymerisieren bzw. zu einem 1,2,4,5-Tetraoxolan dimerisieren oder in einer weiteren Cycloaddition ein Sekundär-Ozonid bilden. Das Sekundär-Ozonid kann anschließend oxidativ zu einer Carbonsäure oder reduktiv zu einem Aldehyd aufgearbeitet werden. Der Aldehyd kann weiter bis zum Alkohol reduziert werden (Vollhardt/Scholer, 1995).

Ein Nachteil des Verfahrens besteht darin, dass aus dem Carbonyloxid nicht nur das Sekundär-Ozonid gebildet wird, das zu den erwünschten Produkten aufgearbeitet werden kann, sondern auch eine Reihe von Nebenprodukten, darunter das Tetraoxolan-Dimer, ein als Hydroperoxid bezeichnetes Addukt aus Ozonid und Wasser sowie daraus entstehendes Wasserstoffperoxid. Darüber hinaus ist die Entstehung und Ansammlung von Sekundär-Ozonid mit Hinblick auf die Sicherheit der Reaktionsführung bedenklich, da derartige Verbindungen als explosiv bekannt sind.

Die Entstehung der weiteren Nebenprodukte verringert die Ausbeute der Reaktion, zumal das entstehende Wasserstoffperoxid auch in der Lage ist, Produkte nachfolgender Schritte zu oxidieren und auf diese Weise zusätzliche Nebenprodukte zu bilden. Entstehende unerwünschte Carbonylverbindungen können zur Bildung zahlreicher unerwünschter Kondensationsprodukte führen.
Pryor et al. in Chem Res Toxicol. 1991 May-Jun;4(3):341-8 offenbaren Produkte der Reaktionen von Ozon mit wäßrigen Emulsionen von ungesättigten Fettsäuren und mit aus Phosphatidylcholin Estern aufgebauten Liposomen.

Vor diesem Hintergrund besteht die der Erfindung zu Grunde liegende Aufgabe darin, ein verbessertes Verfahren bereitzustellen, bei dem das Auftreten von gefährlichen Sekundär-Ozoniden und störenden Nebenprodukten vermindert und die Ausbeute der Reaktion dementsprechend verbessert ist.

Weiterhin liegt der vorliegenden Erfindung die Aufgabe zu Grunde, ein Verfahren zu entwickeln, bei dem die reaktiven Sauerstoffspezies in einer Reaktionsmischung umfassend Ozonolyseprodukte von ungesättigten Fettsäuren und deren Derivaten ohne Verbrauch von Reduktionsäquivalenten abgebaut werden.

Weiterhin liegt der vorliegenden Erfindung die Aufgabe zu Grunde, ein Verfahren zu entwickeln, bei dem der Abbau der reaktiven Sauerstoffspezies in einer Reaktionsmischung umfassend Ozonolyseprodukte von ungesättigten Fettsäuren und deren Derivaten durch einen alternativen Katalysator geleistet wird, der gegenüber bisherig verwendeten Katalysatoren verbesserte Eigenschaften aufweist.

Weiterhin liegt der vorliegenden Erfindung die Aufgabe zu Grunde, ein Verfahren zu entwickeln, bei dem die Behandlung einer Reaktionsmischung umfassend Ozonolyseprodukte von ungesättigten Fettsäuren und deren Derivaten zur Verringerung der Konzentration von Sekundär-Ozoniden unter neutralen Bedingungen erfolgt, um durch extreme pH-Werte begünstigte Nebenreaktionen aldehydischer Ozonolyseprodukte zu vermeiden.

Eine weitere der Erfindung zu Grunde liegende Aufgabe besteht darin, das Verfahren derart zu verbessern, dass es zu einem beschleunigten Abbau von reaktiven Sauerstoffspezies kommt, ohne dass dies einen Wechsel des Lösungsmittels erfordert.

Diese und weitere Aufgaben werden durch den Gegenstand der vorliegenden Erfindung und insbesondere auch durch den Gegenstand der beigefügten unabhängigen Ansprüche gelöst, wobei sich Ausführungsformen aus den Unteransprüchen ergeben.

Erfindungsgemäß wird die Aufgabe in einem ersten Aspekt gelöst durch ein Verfahren umfassend die Schritte:
a) Ozonolyse einer ungesättigten Fettsäure oder eines Derivates davon in einer organischen Phase umfassend ein organisches Lösungsmittel, und
b) Kontaktieren der organischen Phase mit einer wässrigen Phase umfassend Katalase und bevorzugt einen Puffer,
wobei die ungesättigte Fettsäure oder das Derivat davon eine lineare Kette mit wenigstens acht Kohlenstoffatomen aufweist und wobei es sich bei der ungesättigten Fettsäure oder dem Derivat davon um eine Verbindung der Formel (I)

R¹-(CH₂)ₓ-CH=CH-(CH₂)_{y}-COR² (I),

handelt
wobei R¹ aus der Gruppe ausgewählt ist, die Wasserstoff, HO-, HS-, CHO-, R²OC-, unsubstituiertes oder substituiertes, bevorzugt halogeniertes, Alkyl, Cycloalkyl, Heterocycloalkyl, Aryl und Heteroaryl umfasst,
wobei R² aus der Gruppe ausgewählt ist, die HO-, substituiertes oder unsubstituiertes Alkoxy, Alkenoxy, Alkinoxy und Cycloalkoxy mit einem bis sieben Kohlenstoffatomen, -NR³R⁴, Aryloxy und Benzyloxy umfasst,
wobei R³ und R⁴ jeweils und unabhängig voneinander aus der Gruppe ausgewählt ist, die Wasserstoff, Alkyl, bevorzugt Methyl oder Ethyl, Alkenyl, Alkinyl, Aryl und Benzyl umfasst,
und wobei x und y jeweils und unabhängig voneinander 0 oder eine größere ganze Zahl sind.

In einer ersten Ausführungsform des ersten Aspektes ist das organische Lösungsmittel ein organisches Lösungsmittel, das in Anwesenheit von einer wässrigen Phase bei Raumtemperatur eine von der wässrigen Phase separate organische Phase ausbildet.

In einer zweiten Ausführungsform, die eine Ausführungsform der ersten Ausführungsform darstellt, erfolgt Schritt a) in Anwesenheit einer wässrigen Phase.

In einer dritten Ausführungsform, die eine Ausführungsform der zweiten Ausführungsform darstellt, wird vor Schritt b) die wässrige Phase aus Schritt a) entfernt.

In einer vierten Ausführungsform, die eine Ausführungsform der vierten Ausführungsform darstellt, ist R¹ Wasserstoff, R² ist HO-, Methoxy oder Ethoxy, und die Summe von x und y beträgt neun oder mehr.

In einer fünften Ausführungsform des ersten Aspektes, die auch eine Ausführungsform der ersten bis fünften Ausführungsform darstellt, ist die Fettsäure bzw. das Derivat aus der Gruppe ausgewählt, die Ölsäure und Erucasäure und deren Ester, bevorzugt Methyl- oder Ethylester, umfasst.

In einer sechsten Ausführungsform des ersten Aspektes, die auch eine Ausführungsform der ersten bis sechsten Ausführungsform darstellt, ist das organische Lösungsmittel aus der Gruppe ausgewählt, die R⁵R⁶R⁷C-O-R⁸ oder intramolekulare Ether davon umfasst, wobei R⁵, R⁶ und R⁷ jeweils und unabhängig voneinander aus der Gruppe ausgewählt sind, die Wasserstoff, ein Alkyl oder Cycloalkyl mit einem bis sieben Kohlenstoffatomen, sowie unsubstituiertes und substituiertes Aryl umfasst, wobei R⁸ aus der Gruppe ausgewählt ist, die Wasserstoff, ein Alkyl, Cycloalkyl, Hydroxyalkyl und Alkoxyalkyl mit einem bis sieben Kohlenstoffatomen sowie unsubstituiertes oder substituiertes Aryl und Benzyl umfasst, und wobei bevorzugt wenigstens einer der Reste R⁵, R⁶ und R⁷ Methyl ist.

In einer siebten Ausführungsform, die eine Ausführungsform der siebten Ausführungsform darstellt, handelt es sich bei dem organischen Lösungsmittel um eine Verbindung der Formel R⁵R⁶R⁷C-OH, und R⁵, R⁶ und R⁷ sind jeweils und unabhängig voneinander aus der Gruppe ausgewählt, die Methyl, Ethyl, Propyl, Butyl und Pentyl umfasst.

In einer achten Ausführungsform, die eine Ausführungsform der achten Ausführungsform darstellt, handelt es sich bei dem organischen Lösungsmittel um 2-Methyl-2-butanol.

In einer neunten Ausführungsform des ersten Aspektes, die auch eine Ausführungsform der ersten bis neunten Ausführungsform darstellt, beträgt der Volumenanteil der wässrigen Phase beim Kontaktieren der organischen Phase am Gesamtvolumen beider Phasen mehr als 0 bis 50, bevorzugter 5 bis 30, noch bevorzugter 15 bis 25 Prozent.

In einer zehnten Ausführungsform des ersten Aspektes, die auch eine Ausführungsform der ersten bis zehnten Ausführungsform darstellt, ist bei Schritt a) Katalase anwesend.

In einer elften Ausführungsform des ersten Aspektes, die auch eine Ausführungsform der ersten bis elften Ausführungsform darstellt, handelt es sich bei der Katalase um Rinderleberkatalase oder eine Variante davon.

In einer zwölften Ausführungsform des ersten Aspektes, die auch eine Ausführungsform der ersten bis zwölften Ausführungsform darstellt, beträgt der pH-Wert der wässrigen Phase in Schritt b) vor dem Kontaktieren mit der organischen Phase 3 bis 11, bevorzugt 5 bis 8, noch bevorzugter 6,5 bis 7,5.

Erfindungsgemäß wird die Aufgabe in einem zweiten Aspekt gelöst durch die Verwendung von Katalase zur Entfernung reaktiver Sauerstoffspezies, bevorzugt organischer reaktiver Sauerstoffspezies, aus einem Reaktionsgemisch umfassend Ozonolyseprodukte einer ungesättigten Fettsäure oder eines Derivates, wie in ersten Aspekt definiert davon.

In einer ersten Ausführungsform des zweiten Aspektes handelt es sich bei der ungesättigten Fettsäure oder dem Ester davon um eine Verbindung der Formel

R¹-(CH₂)ₓ-CH=CH-(CH₂)_{y}-COR² (I),

wobei R¹ aus der Gruppe ausgewählt ist, die Wasserstoff, HO-, HS-, CHO-, R²OC-, unsubstituiertes oder substituiertes, bevorzugt halogeniertes, Alkyl, Cycloalkyl, Heterocycloalkyl, Aryl und Heteroaryl umfasst,
wobei R² aus der Gruppe ausgewählt ist, die HO-, substituiertes oder unsubstituiertes Alkoxy, Alkenoxy, Alkinoxy und Cycloalkoxy mit einem bis sieben Kohlenstoffatomen, -NR³R⁴, Aryloxy und Benzyloxy umfasst,
wobei R³ und R⁴ jeweils und unabhängig voneinander aus der Gruppe ausgewählt ist, die Wasserstoff, Alkyl, bevorzugt Methyl oder Ethyl, Alkenyl, Alkinyl, Aryl und Benzyl umfasst,
und wobei x und y jeweils und unabhängig voneinander 0 oder eine größere ganze Zahl sind.

In einer zweiten Ausführungsform des zweiten Aspektes, die auch eine Ausführungsform der ersten Ausführungsform darstellt, enthält das Reaktionsgemisch weiter ein organisches Lösungsmittel, bevorzugt eines, das in Anwesenheit von einer wässrigen Phase bei Raumtemperatur eine von der wässrigen Phase separate organische Phase ausbildet,
wobei das organische Lösungsmittel aus der Gruppe ausgewählt ist, die R⁵R⁶R⁷C-O-R⁸ oder intramolekulare Ether davon umfasst, wobei R⁵, R⁶ und R⁷ jeweils und unabhängig voneinander aus der Gruppe ausgewählt sind, die Wasserstoff, ein Alkyl oder Cycloalkyl mit einem bis sieben Kohlenstoffatomen, sowie unsubstituiertes und substituiertes Aryl umfasst, wobei R⁸ aus der Gruppe ausgewählt ist, die Wasserstoff, ein Alkyl, Cycloalkyl, Hydroxyalkyl und Alkoxyalkyl mit einem bis sieben Kohlenstoffatomen sowie unsubstituiertes oder substituiertes Aryl und Benzyl umfasst, und wobei bevorzugt wenigstens einer der Reste R⁵, R⁶ und R⁷ Methyl ist.
wobei bevorzugt wenigstens einer der Reste R⁵, R⁶ und R⁷ Methyl ist,
und wobei es sich bei dem organischen Lösungsmittel noch bevorzugter um 2-Methyl-2-butanol handelt.

Die Erfinder der vorliegenden Erfindung haben überraschend gefunden, dass die Effizienz und Ausbeute der Ozonolyse von ungesättigten Fettsäuren und deren Derivaten sowie die Reinheit der Reaktionsprodukte durch die Anwesenheit von Katalase in der Reaktionsmischung nach Durchführung der Ozonolyse gesteigert werden kann. Die Erfinder haben weiterhin überraschend gefunden, dass die Aktivität von Katalase mit dem bei der Ozonolyse entstandenen Reaktionsgemisch kompatibel ist. Schließlich haben die Erfinder überraschend gefunden, dass die Anwesenheit von Katalase in der Ozonolyse-Reaktionsmischung die Konzentration von reaktiven Sauerstoffspezies wie Sekundär-Ozonid sowie die Bildung weiterer Nebenprodukte verringert und die Entstehung der Carbonylprodukte beschleunigt.

In einer bevorzugten Ausführungsform bedeutet der Begriff "Ozonolyse", wie hierin verwendet, ein Verfahren, bei dem eine C=C-Doppelbindung eines Kohlenwasserstoffes, bevorzugterweise einer ungesättigten Fettsäure oder eines Derivates davon, durch Einwirkung von Ozon auf das Molekül oxidativ unter Bildung von Carbonylprodukten gespalten wird. Die zur Durchführung der Ozonolyse geeigneten Verfahren, Agenzien und Geräte sind dem Fachmann aus dem Stand der Technik bekannt (Baily, 1978). Sie wird üblicherweise in Alkoholen als Lösungsmittel durchgeführt, wobei das Reaktionsgemisch weiterhin bezogen auf die Gesamtmenge an Lösungsmittel mindestens 0,5 Gewichtsprozent Wasser enthält. Üblicherweise liegt die ungesättigte Fettsäure oder ihr Derivat in einer Konzentration von 0,1 bis 1 mol/L vor. Die Ozonolyse wird bevorzugt bei Temperaturen von 0 bis 40 °C durchgeführt, noch bevorzugter bei Temperaturen von 10 bis 35 °C, und besonders bevorzugt bei Temperaturen von 20 bis 30 °C. Üblicherweise wird zur Erzeugung des Ozons ein Ozongenerator verwendet, der als Speisegas technische Luft oder eine Gemisch aus Kohlendioxid und Sauerstoff einsetzt. Aus dem Sauerstoff wird das Ozon durch stille elektrische Entladung hergestellt. Dabei werden Sauerstoffradikale gebildet, die mit weiteren Sauerstoffmolekülen Ozonmoleküle bilden.

Eine besondere Stärke des erfindungsgemäßen Verfahrens besteht darin, dass eine Verbesserung des Verfahrens durch den Zusatz einer enzymatischen Aktivität, nicht durch die Verwendung aggressiver oder gefährlicher Verbindungen bzw. Reaktionsbedigungen erzielt werden kann. Die Verwendung von aktiven Enzymen in der organischen Synthese ist bis heute überwiegend auf Verfahren im kleinen Maßstab beschränkt. Enzyme gelten nicht nur als hochpreisige Agenzien mit geringer Substratspezifität, sondern sind aufgrund verschiedener Effekte mit zahlreichen in der organischen Synthese gängigen Lösungsmitteln inkompatibel, deren Verwendung in vielen Fällen immer auch eine Voraussetzung für die wirtschaftliche Aufarbeitung der gewünschten Reaktionsprodukte ist. Als Polypeptide weisen Enzyme in der Regel eine mehr oder weniger globuläre Struktur auf, die insbesondere dadurch stabilisiert ist, das aliphatische Aminosäureseitenketten im Inneren einen hydrophoben Kern bilden, wohingegen an der Oberfläche des Enzyms hydrophile, polare Aminosäureseitenketten vorherrschen. Dementsprechend benötigt das Enzym zu seiner Stabilisierung eine Hydrathülle aus Wassermolekülen und neigt zur Denaturierung, wenn es in Kontakt mit einem Lösungsmittel steht, das mit ihm um wenige verfügbare Wassermoleküle konkurriert. Bei biphasischen Systemen umfassend eine wässrige Phase und eine organische Phase kommt es häufig zu Grenzflächeneffekten, die eine Absorption und Inaktivierung der Enzymmoleküle an der Phasengrenze umfassen können. Die Anwesenheit von organischen Lösungsmitteln kann weiterhin die kinetischen Eigenschaften des Enzyms, d. h. seine Aktivität und seine Selektivität beeinflussen, beispielsweise durch reversible oder irreversible Denaturierung des Enzyms oder durch Veränderung seiner Selektivität oder durch Beeinflussung des Reaktionsgleichgewichtes. Auch ist die verlässliche Einstellung eines bestimmten pH-Bereiches, der für die Aktivität von Enzymen essentiell ist, in Gegenwart von organischen Lösungsmitteln schwierig (Wong, 1994).

In einer bevorzugten Ausführungsform wird unter dem Begriff "Katalase", wie hierin verwendet, ein enzymatisch aktives Polypeptid verstanden, dass die Zersetzung von Wasserstoffperoxid zu Wasser und Sauerstoff katalysiert. Eine Katalyse liegt in einer bevorzugten Ausführungsform dann vor, wenn die Zersetzung von Wasserstoffperoxid in Gegenwart nachweisbarer Mengen des katalytisch wirksamen Polypeptides schneller abläuft als in dessen Abwesenheit. In einer bevorzugten Ausführungsform handelt es sich bei dem enzymatisch aktiven Polypeptid um ein multifunktionelles Enzym, das neben der Katalaseaktivität eine oder mehr als eine weitere Aktivität aufweist. Beispiele für erfindungsgemäß verwendbare Polypeptide umfassen Rinderleberkatalase (Datenbankcode NP_001030463), Meerrettich-Peroxidase, zum Beispiel Datenbankcode, CAA00083.1, sämtliche weitere im Stand der Technik beschriebenen Katalasen (Abad-Zapatero, 1992; Abe *et al.* 1973, Altomare et al., 1974, Baird *et al*, 1987), Balasubramaniam *et al*., 1987; Barlow & Margoliash, 1969; Beers & Sizer, 1952; Beyer & Fridovich, 1985; Boismenu *et al*., 1989; Browett & Stillman, 1980; Chance, 1951; Chance, 1955, Chance, 1973, Chance *et al*., 1973; Chatterjee *et al*., 1989; Ch *et al*., 1975; Corrall *et al*., 1974; Darr & Fridovich, 1985; Davison *et al*., 1986; Deisseroth & Dounce, 1969; Diesseroth *et al*., 1967; Eglinton *et al*., 1983; Escobar *et al*., 1990; Evans & Recheigl, 1967; Feinstein *et al*., 1967; Feinstein *et al*, 1967; Fita & Rossman, 1985; Furuta *et al*., 1974; Ghadermarzi & Moosavi-Movahedi, 1996; Goldacre & Galston, 1953; Goldstein, 1968; Goth, 1989; Goth, 1991; Gregory & Fridovich, 1974; Gadermarzi & Moosavi-Movahedi, 1996; Goldacre & Galston, 1953; Goldstein, 1968; Goth, 1989; Goth, 1991; Gregory & Fridovich, 1974; Haining & Legan, 1972; Halliwell, 1973; Heidrich, 1968, Higashi *et al*., 1974; Holmes, 1972; Holmes & Masters, 1972; Johansson & Borg, 1988; Jones & Masters, 1975; Jones, 1973; Jones & Middlemiss, 1972; Kirkman & Gaetani, 1984; Kiselev *et al*., 1967; Kremer, 1970; Kroll *et al*., 1989; Kyle *et al*., 1987; Lanir & Schejter, 1975; Lardinois, 1995; Lee *et al*., 1987; Liebermann & Ove, 1958; Loewen & Seitala, 1987; Longley, 1967; Maehly & Chance, 1954; Maimoni *et al*., 1999; Margoliash & Novogorodsky, 1958; Marklund, 1972, Mastersc, 1986; McPherson & Rlch, 1973; Mitchel & Anderson, 1965; Miyahara *et al*., 1978; Murphy *et al*., 1967; Madler *et al*., 1986; Nelson & Niesow, 1972M Nicholls *et al*., 1963; Orr, 1967a; Orr, 1967b; Oshino *et al*., 1973; Percy *et al*., 1990; Perrin *et al*., 1990; Pitts, 1992; Poznansky *et al*., 1974; Pritchard & Hudson, 1967; Roth & Jensen, 1967; Rossman & Labaw, 1967; Sakamoto & Highashi, 1974; Samejima; Kita, 1969; Samejima *et al*., 1962; Schroeder *et al*., 1969; Scibior & Czeczot, 2006; Scott *et al*., 1991; Seah & Kaplan, 1973; Seah *et al*., 1973; Sharma *et al*., 1989; Shimiza *et al*., 1984; Spreti *et al*., 1995; Sumner & Dounce, 1937; Sumner *et al*., 1940; Sund *et al*., 1967; Tanaka *et al*., 1991; Tanford & Lovrien, 1962; Tauber & Petit, 1952; Tudhope, 1967; Vuilaum *et al*., 1988; Wheeler *et al*., 1990; Whiteside *et al*., 1988; Yasukochi *et al*., 1972; Zidoni & Kremer, 1974) und deren Varianten.

Für den Fall, dass für die Aktivität der Katalase neben Wasserstoffperoxid noch ein weiteres Substrat und/oder einen oder mehr als einen Cofaktor erforderlich ist, umfasst die Katalase dieses. Z.B. ist im Falle der Verwendung von Meerrettich-Peroxidase die Anwesenheit von 2,2'-azino-bis(3-ethylbenzthiazolin-6-sulfonsäure) erforderlich.

Die verwendete Katalase kann der Reaktionsmischung in verschiedener Form und in verschiedenen Aufreinigungsstufen, von einer unverarbeiteten Lösung oder einem Pellet Katalaseaktivität aufweisender Zellen bis zu einem vollständig aufgereinigten Enzym zugesetzt werden. In einer bevorzugten Ausführungsform handelt es sich bei der Katalase um ein mit einer ganzen, entweder lebensfähigen oder lysierten Zelle assoziiertes Polypeptid, beispielsweise um eines, das von einem prokaryontischen Ganzzellkatalysator, bevorzugt bakterieller Natur, exprimiert wird. Dabei muss eine Zelle, wenn sie als Ganzzellkatalysator verwendet wird, die Katalase nicht in ihrem Inneren exprimieren, sondern auch eine an der Außenmembran lokalisierte Katalase ist möglich, beispielsweise ein Fusionsprotein mit einem endogenen Außenmembranprotein. In einer weiteren bevorzugten Ausführungsform handelt es sich um das Lysat einer Zelle, bevorzugt bakterieller Natur, die die Katalase im Zellinneren exprimiert. In einer weiteren Ausführungsform handelt es sich um eine Lösung oder ein Lyophilisat eines isolierten, bevorzugt zu wenigstens 60, 70, 80, 90 oder 95, besonders bevorzugt 100 % reinen Polypeptides. Präparationen derartiger Zellen oder Polypeptide sind im Handel erhältlich oder für den Fachmann anhand von biochemischen Standardverfahren herstellbar.

Die Konzentration der Katalase beträgt in einer bevorzugten Ausführungsform 0,001 bis 1, noch bevorzugter 0,01 bis 0,5, am bevorzugtesten 0,025 bis 0,1 Gewichtsprozent der wässrigen Phase bei Schritt b).

Die Abfolge und Ausführung der Schritte a) und b) kann in sämtlichen denkbaren Variationen erfolgen, solange die Katalase mit den Produkten der Ozonolyse der Fettsäure oder des Derivates davon in Kontakt kommt. Während die Anwesenheit einer wässrigen Phase umfassend Katalase bei Schritt b) essentiell ist, ist sie bei Schritt a) optional. So kann die gleiche wässrige Phase bei Schritt a) und b) vorhanden sein, es ist jedoch auch möglich, Schritt a) in Anwesenheit einer wässrigen Phase durchzuführen, diese jedoch zu entfernen und für Schritt b) eine andere wässrige Phase zu verwenden. Ebenso ist es möglich, während oder vor Schritt a) eine wässrige Phase mit oder ohne Katalase zur organischen Phase zu geben und die Katalase nach Abschluss der Ozonolyse in Schritt a) in die wässrige Phase zu geben. In einer besonders bevorzugten Ausführungsform wird die Katalase erst nach der Ozonolyse zur organischen Phase gegeben.

Dem Fachmann sind zahlreiche Verfahren und Protokolle bekannt, nach denen katalytisch aktive Katalasen aus Wildtypzellen oder rekombinanten Zellen aufgereinigt werden können, siehe beispielsweise Yumoto *et al*., 2000.

Als Edukt für die Ozonolyse kommen sämtliche denkbaren Fettsäuren und deren Derivate in Frage, sowohl natürlich vorkommende als auch künstlich synthetisierte, solche mit einer geraden als auch einer ungeraden Zahl von Kohlenstoffatomen und verzweigte wie auch unverzweigte, insbesondere Undecylensäure, Myristoleinsäure, Palmitoleinsäure, Petroselinsäure, Ölsäure, Elaidinsäure, Vaccensäure, Gadoleinsäure, Icosensäure, Cetoleinsäure, Erucasäure, Nervonsäure und deren Derivate, bevorzugt deren Methylester. Weiterhin können auch mehrfach ungesättigte Fettsäuren eingesetzt werden, beispielsweise Linolsäure, Linolensäure, Calendulasäure, Punicinsäure, Elaeosterarinsäure, Arachidonsäure, Timnodonsäure, Clupanodonsäure und Cervonsäure und deren Derivate, bevorzugt deren Methylester. In einer bevorzugten Ausführungsform bedeutet, dass "die Fettsäure oder das Derivat davon eine lineare Kette mit wenigstens acht Kohlenstoffatomen aufweist", wie hierin verwendet, dass das Kohlenstoffatom der Carboxylgruppe Teil, bevorzugt am Ende, einer linearen Kette mit wenigstens acht Kohlenstoffatomen ist, die Verzweigungen aufweisen darf, sofern die Länge von acht Kohlenstoffatomen über die längste mögliche lineare Kette im Kohlenstoffgerüst gegeben ist.

In einer bevorzugten Ausführungsform handelt es sich bei den als "Alkoxy", "Alkenoxy" bzw. "Alkinoxy" bezeichneten Substituenten, wie hierin verwendet, um Substituenten der Formel R-O-, wobei es sich bei R um ein Alkyl, Alkenyl bzw. Alkinyl handelt.

Ein besonderer Vorteil des erfindungsgemäßen Verfahrens besteht darin, dass Ozonolyse und Entfernung der reaktiven Sauerstoffspezies in mit beiden Reaktionen kompatiblen Lösungsmitteln bzw. Lösungsmittelgemischen, d.h. in einer Eintopf-Reaktion durchgeführt werden können, so dass ein aufwändiger Wechsel des Lösungsmittel unterbleiben kann. In einer bevorzugten Ausführungsform wird Schritt b) dementsprechend ohne Wechsel des Lösungsmittels oder ohne Abtrennung von Bestandteilen der Reaktionsmischung aus Schritt a) im gleichen Reaktionsgefäß durchgeführt.

In einer besonderen Ausführungsform beträgt der Volumenanteil der wässrigen Phase beim Kontaktieren der organischen Phase am Gesamtvolumen beider Phasen mehr als 0,5, 1, 5, 10, 15, 20, 25 oder mehr als 0 bis 50, mehr als 1 bis 40, mehr als 2,5 bis 25, bevorzugter 5 bis 30, noch bevorzugter 15 bis 25 Prozent.

Grundsätzlich kann für Schritt a) eine Vielzahl von Lösungsmitteln Verwendung finden, beispielsweise Aliphaten wie Hexan oder Cyclohexan, Aromaten alkylierte Aromaten wie Benzol, Toluol oder Xylol, halogenierte Aliphaten oder Aromaten wie Dichlormethan bzw. Chlorbenzol, Ketone, beispielsweise Aceton oder Cyclohexanon, Alkohole und deren Ether wie 2-Butanol, 2-Methyl-2-propanol oder Methyl-tert.-butylether, Carbonsäuren und deren Ester wie Essigsäure oder Ethylacetat, Sulfoxide wie Dimethylsulfoxid, und Heterocyclen wie Tetrahydrofuran oder Dioxan. Besonders bevorzugte Lösungsmittel umfassen sekundäre und tertiäre Alkohole, darunter besonders 2-Propanol und tert.-Butanol. Handelt es sich bei dem Lösungsmittel um ein Lösungsmittel der Formel R⁵R⁶R⁷C-O-R⁸, so können die Reste R⁵, R⁶, R⁷ und R⁸ jeweils paarweise und unabhängig voneinander Alkylenbrücken der Formel -(CH₂)_{z}- bilden, wobei z derart gewählt ist, dass der entsprechende Ring wenigstens 3, bevorzugt wenigstens 5 Glieder hat. Weiterhin können die Reste auch intramolekulare Ether bilden, die formell das Ergebnis der Kondensation zweier hydroxyhaltiger Reste sind. Beispielsweise ist ein erfindungsgemäßes Lösungsmittel für den Fall, dass R⁵ = R⁶ = Methyl, R⁷ Hydroxypropyl und R⁸ Wasserstoff ist, die Verbindung mit der Formel

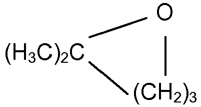

Die Abtrennung der organischen Lösungsmittel, insbesondere wenn diese ohne Zutun eine von der wässrigen Phase separate Phase ausbilden, ist für den Fachmann einfach und erfolgt beispielsweise durch Dekantieren, Verwendung eines Scheidetrichters und dergleichen.

In einer bevorzugten Ausführungsform wird unter dem Begriff "eine von der wässrigen Phase separate organische Phase", wie hierin verwendet, eine bei Raumtemperatur überwiegend, bevorzugt zu wenigstens 90 Volumenprozent, aus organischen Lösungsmitteln bestehende homogene flüssige Phase verstanden, die sich nach Kontaktieren mit einer wässrigen Phase ohne weiteres Zutun von dieser absetzt, wie es bevorzugt rein visuell feststellbar ist, so dass es nach Einstellung des Gleichwichts zur Bildung zweier getrennter Phasen kommt.

Den Schritten a) und b) wird sich in der Regel eine Weiterverarbeitung des von reaktiven Sauerstoffspezies befreiten Ozonolyseproduktes anschließen. Möglich ist beispielsweise die reduktive Aminierung, bei der ein Aldehyd oder Keton aminiert wird, d.h. im Falle eines ω-Oxo-Carbonsäure-Ozonolyseproduktes kommt es zur Bildung von einer ω-Aminocarbonsäure. Eine solche reduktive Aminierung wird bevorzugt mit Hilfe eines Raney-Nickel-Katalysators und Wasserstoff durchgeführt, wie im Stand der Technik beschrieben ist, beispielsweise in Chan *et al*., 1994. Alternativ kann eine Weiteroxidation zur α,ω-Dicarbonsäure sinnvoll sein.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

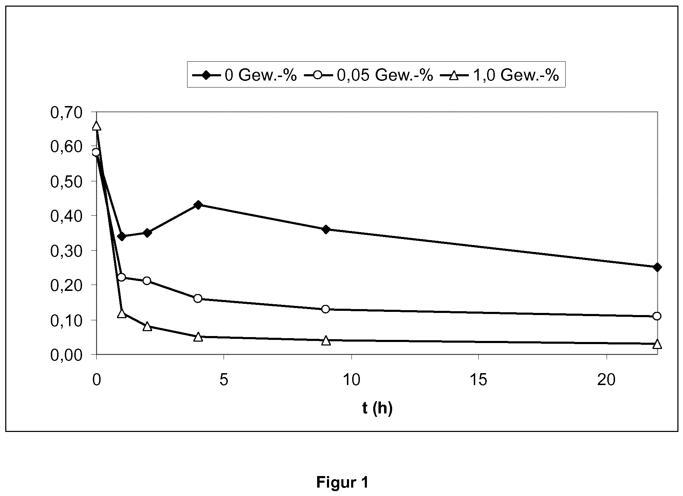

Fig. 1 zeigt die Abnahme der Konzentration von reaktiven Sauerstoffspezies nach der erfindungsgemäßen Behandlung einer Lösung von Ozonolyseprodukten mit verschiedenen Mengen lyophilisierter Rinderleberkatalase, wie in Beispiel 1 beschrieben ist. Die y-Achse zeigt die Menge an reaktiven Sauerstoffspezies in Gewichtsprozent.

### Beispiel 1: Ozonolyse von Methyl-cis-13-docosenoat (Methylerucat) mit anschließender Katalase-Behandlung

Alle Chemikalien und Lösungsmittel wurden in höchstmöglicher Qualität (mindestens p.a.) im freien Handel bezogen und ohne weitere Aufreinigung bzw. Behandlung eingesetzt, Wasser nur in entmineralisierter Qualität. Phosphat-Pufferlösung (pH = 7,0) wurde bei Bernd Kraft GmbH (Duisburg, Deutschland) erworben, gefriergetrocknete Rinderleberkatalase bei Sigma-Aldrich (St. Louis, USA). Die Fettsäureester Methyl-*cis*-9-octadecenoat (Methyloleat) und Methyl-*cis*-13-docosenoat (Methylerucat) wurden als ≥ 95 proz. Materialien von Croda (Gouda, Niederlande) bezogen. Laut GC-Analytik (unkorrigiert) betrug die Reinheit des C18:1- bzw. C22:1-Methylesters 95,2 bzw. 95,0 %. Im Falle des Methyloleats lagen als Verunreinigungen 3,6 % an C18:2-Methylestern sowie summiert 1,2% der gesättigten Methylester Methylhexadecanoat, -octadecanoat und -eicosanoat (Methylpalmitat, -stearat und -arachidat) vorl, d.h. 98,8 % ungesättigte, d.h. spaltbare Methylester. Im Falle des Methylerucats umfassten die Verunreinigungen 1,3 % an C22:2-Methylestern, 0,7 % an C20:1-Methylestern und 1,0 % an C24:1-Methylestern sowie summiert 2,0% der gesättigten Methylester Methyleicosanoat, -docosanoat und -tetracosanoat (Methylarachidat, -behenat und -lignocerat), d.h. 98,0 % des Ausgangsmaterials waren ungesättigte, spaltbare Methylester.

Ein 0,912 molales Zweiphasengemisch aus einer Lösung von 80.0 g Methyl-*cis*-13-docosenoat (Methylerucat) in 160 mL 2-Methyl-2-butanol und 30 mL Wasser wird in einer kegelförmigen 250 mL-Dreihalsvorlage mit Bodenauslass (Teflonhahn) bereitgestellt. Über letzteren ist eine Schlauchpumpe angeschlossen, über den mittleren (NS29-) Normschliff eine mit Raschigringen (6 x 6 x 0,65 mm) gefüllte Rieselbett-Glaskolonne (800 x 30 mm) mit unten und oben liegender Gaszu- sowie oben liegender Gasableitung. Über den einen seitlichen (NS14-) Normschliff ist ein Innenthermometer angeschlossen, der andere Normschliff ist frei (Probennahmestelle). Über die Pumpe wird das Zweiphasengemisch nun bei Raumtemperatur über die Kolonne im Kreislauf gefahren (Flussrate: 120 mL/ min). Im Gegenstrom zum Lösungsfluss wird von unterhalb der Kolonne ein Reaktivgas aus einem O₂/O₃-Gemisch aus einem Ozongenerator geleitet (1500 Norm-mL/min O₂-Strom, maximale Leistung Generator). Von oberhalb der Kolonne wird CO₂ als Träger- bzw. Spülgas in den O₃-abgereicherten O₂-Abfluss eingemischt (ebenfalls 1500 Norm-mL/min). Der Abgasstrom fließt über eine zwischengeschaltete Kühlfalle mit Eiskühlung durch 100 mL einer 5 Gew.-proz. wässrigen Kaliumiodid-Lösung als Ozon-Indikator. Zur Probennahme werden einige Tropfen des Reaktionsgemischs per Pipette entnommen, in 2 mL Ethylacetat/Wasser (1:1) aufgenommen, kurz ausgeschüttelt, und die organische Phase dann per GC analysiert (15 m DB-1-Säule).

Nach 65 min tritt vollständiges Mischen der beiden Phasen ein, nach 110 min ist die Reaktion beendet (> 99,5 % Umsatz bzw. < 0,5 % verbleibendes Edukt), worauf die Pumpe ausgeschaltet und die Durchleitung der beiden Gase beendet wird. Die Temperatur des Reaktionsgemischs steigt während der gesamten Zeit um deutlich weniger als 10 °C an.

Als Produkte in der erhaltenen Reaktionslösung liegen laut GC-Analyse Nonanal und Methyl-13-oxodecanoat zu gleichen Teilen in einer relativen Reinheit von > 95 % vor; es finden sich die Produkte der weiteren Oxidation dieser beiden Verbindungen, Nonansäure (Pelargonsäure) bzw. 13-Hydroxy-13-oxomethyltridecanoat (Methylbrassylat), zu je < 5 % in der Lösung.

Die Produktlösung wird aus der Apparatur abgelassen, die geleerte Apparatur mit je 160 mL 2-Methyl-2-butanol und Tetrahydrofuran gespült und die erhaltene Lösung in drei gleiche Teile zu je 200 mL aufgeteilt. Zu jeder der drei Lösungen wird nun 100 mL einer frisch zubereiteten wässrigen Lösung von Rinderleberkatalase in Phosphat-Pufferlösung (pH = 7,0) gegeben, wobei die Endkonzentration der Rinderleberkatalase in der wässrigen Lösung im ersten Fall 0 Gew.-% (Blindprobe), im zweiten Fall 0,05 Gew.-% , und im dritten Fall 1,0 Gew.-% beträgt. Alle drei Lösungen werden noch einmal mit 100 mL Pufferlösung eingespült. Die so erhaltenen Zweiphasengemische werden bei Raumtemperatur stark gerührt, und nach 1, 2, 4, 9 und 22 h werden je 40 mL Probe entnommen und auf den Gehalt an reaktivem Sauerstoff analysiert.

Dazu wird die Probe unter Schutzgas (N₂) in Gegenwart von überschüssigem Natriumiodid am Rückfluss gekocht. Nach dem Abkühlen wird das entstandene Iod mit einer Thiosulfat - Lösung potentiometrisch titriert: Zur Probe der im Erlenmeyerkolben eingewogenen Probe wird zunächst Trockeneis, anschließend 80 ml 1-Propanol, 4 ml Essigsäure sowie 20 ml isopropanolische Natriumiodid-Lösung hinzugegeben. Nachdem ein Rückflusskühler aufgesetzt wurde, wird Stickstoff über die Kühler eingeleitet. Die Reaktionsmischung wird anschließend eine halbe Stunde bei Raumtemperatur stehen gelassen und dann 10 Minuten lang gekocht. Nach Abkühlung werden ca. 20 ml Wasser durch den Kühler gespült, anschließend wird die Mischung mit 0,01 mol/l Natriumthiosulfat-Lösung wird bis zum Äquivalenzpunkt titriert. Parallel wird auf die gleiche Weise eine Blindwertbestimmung durchgeführt.

Die entsprechenden Ergebnisse sind **Tab. 1** bzw. **Fig. 1** zu entnehmen.

**Tab. 1: Konzentration reaktiver Sauerstoffspezies nach Zugabe verschiedener Mengen Katalase zu einer Reaktionsmischung mit Ozonolyseprodukten und Methylerucat**

| **Laufzeit in h** | **0 Gew.-% lyophilisierte Katalase** | **0,05 Gew.-% lyophilisierte Katalase** | **1,00 Gew.-% lyophilisierte Katalase** |
|---|---|---|---|
| 0 | 0,58 | 0,58 | 0,66 |
| 1 | 0,34 | 0,22 | 0,12 |
| 2 | 0,35 | 0,21 | 0,08 |
| 4 | 0,43 | 0,16 | 0,05 |
| 9 | 0,36 | 0,13 | 0,04 |
| 22 | 0,25 | 0,11 | 0,03 |

Es ist mithin klar erkennbar, dass gegenüber der Blindprobe bereits die Verwendung einer Lösung von 0,05 Gew.-% Rinderleberkatalase bewirkt, dass der Anteil potentiell oxidierend wirkender Bestandteile in der organischen Produktlösung um einen Faktor > 2 abnimmt, bei Verwendung einer Lösung von 1 Gew.-% Rinderleberkatalase resultiert sogar ein Faktor von beinahe 10.

Insbesondere die rasche Abnahme zu Beginn der Behandlung zeigt klar, dass mithilfe einer solchen Behandlung erreicht wird, dass Produktlösungen gegenüber einer weiteren Oxidation sehr labiler Verbindungen wie aliphatischer Aldehyde in diesem Sinne stabilisiert werden können.

### Literaturstellen

Baily, P. S., Ozonation in organic chemistry, Academic: New York, NY, 1978, Vol. 1. Wong, C.-H., Enzymes in synthetic organic chemistry, Elsevier Science Limited, 1994
DE 102011015150.8 (2011): SYNTHESE VON OMEGA-AMINOCARBONSÄUREN UND DEREN ESTERN AUS UNGESÄTTIGTEN FETTSÄUREDERIVATEN Vollhardt/Scholer (1995): Organische Chemie, 2. Auflage, VCH
Yumoto, et al. (2000) Purification and Characterisation of a Catalase from the Facultatively Psychrophilic Bacterium, Vibrio rumoiensis S-1 Exhibiting High Catalase Activity, J. Bact. 182(7), 1903 - 1909
Abad-Zapatero, C.: Crystallization by Centrifugation , Nature 356, 392, 1992
Abe, K., Hiraga, M., and Anan, F.: The Chemical Modification of Beef Liver Catalase. IV. The Properties of Acrylonitrile- or Glyoxal-Treated Products of Catalase , J Biochem (Tokyo) 74, 889, 1973
Altomare, R., Kohler, J., Greenfield, P., and Kittrell, J.: Deactivation of Immobilized Beef Liver Catalase by Hydrogen Peroxide , Biotechnol Bioeng 16, 1659, 1974
Baird, M., Sfeir, G., and Slade-Pacini, C.: Lack of Inhibition of Mouse Catalase Activity by Cimetidine: An Argument Against a Relevant General Effect of Cimetidine Upon Heme Metabolic Pathways , Biochem Pharmacol 36, 4366, 1987
Balasubramanian, P., Schmidt, E., and Bruice, T.: Catalase Modeling. 2.Dynamics of Reaction of a Water-Soluble and Non u-Oxo Dimer Forming Manganese (III) Porphyrin with Hydrogen Peroxide, J Am Chem Soc 109, 7865, 1987
Barlow, G., and Margoliash, E.: Sedimentation Studies on Catalase Complex II Using Photoelectric Scanning Optics , Biochim Biophys Acta Gene Struct. Expr. 188, 159, 1969
Beers, R., and Sizer, I.: A Spectrophotometric Method for Measuring the Breakdown of Hydrogen Peroxide by Catalase , J Biol Chem 195, 133, 1952
Beyer, W., and Fridovich, I.: Pseudocatalase from Lactobacillus plantarum: Evidence for a Homopentameric Structure Containing Two Atoms of Manganese per Subunit , Biochem 24, 6460, 1985
Boismenu, D., Lepine, F., Gagnon, M., and Dugas, H.: Catalase Activity Measurement with the Disk Flotation Method , Anal Biochem 178, 404, 1989
Browett, W., and Stillman, M.: Magnetic Circular Dichroism Studies on the Electronic Configurations of Catalase Compounds I and II , Biochim Biophys Acta 623, 21, 1980
Chan, A. S. C., Chen, C.-C., and Lin, Y.-C. (1994) Catalytic reductive Amination of α-ketocarboxylic acids as a useful route to amino acids, Applied Catalysis A General 119, L1-L5
Chance, B.: The Iron-Containing Enzymes. C. The Enzyme-Substrate Compounds and Mechanism of Action of the Hydroperoxidases, The Enzymes Vol. 2, Pt. 1,J. Sumner and K. Myrback, Academic Press, NY, , 1951
Chance, B., and Maehley, A.: Assay of Catalases and Peroxidases, Meth Enzymol 2, 764, 1955
Chance, B., and Oshino, N.: Analysis of the Catalase-Hydrogen Peroxide Intermediate in Complex Oxidations , Biochem J 131, 564, 1973
Chance, B., Boveris, A., Oshino, N., and Loschen, G.: The Nature of the Catalase Intermediate in the Biological Function, Oxidases and Related Redox Systems Vol. I,T. King, H. Mason, and M. Morrison, University Park Press, Baltimore, USA, 350, 1973
Chatterjee, U., Kumar, A., and Sanwal, G.: Purification and Properties of Goat Liver Catalase - Two pH Optima , Indian J Biochem Biophys 26, 140, 1989
Chu, H., Leeder, J., and Gilbert, S.: Immobilized Catalase Reactor for Use in Peroxide Sterilization of Dairy Products, J Food Sci 40, 641, 1975
Corrall, R., Rodman, H., Margolis, J., and Landau, B.: Stereospecificity of the Oxidation of Ethanol by Catalase , J Biol Chem 249, 3181, 1974
Darr, D., and Fridovich, I.: Inhibition of Catalase by 3,3'-diamine-benzidine , Biochem J 226, 781, 1985
Davison, A., Kettle, A., and Fatur, D.: Mechanism of the Inhibition of Catalase by Ascorbate , J Biol Chem 261, 1193, 1986
Deisseroth, A., and Dounce, A.: Comparison of the Catalytic and Physical Properties of the Components of Lyophilized Beef Erythrocyte Catalase with those of Lyophilized Beef Liver Catalase Components , Arch Biochem Biophys 131, 30, 1969
Diesseroth, A., and Dounce, A.: Nature of the Change Produced in Catalase by Lyophilization, Arch Biochem Biophys 120, 671, 1967
Eglinton, D., Gadsby, P., Sievers, G., Peterson, J., and Thompson, A.: A Comparative Study of the Low-Temperature Magnetic Circular Dichroism Spectra of Horse Heart Metmyoglobin and Bovine Liver Catalase Derivatives , Biochim Biophys Acta 742, 648, 1983
Escobar, L., Salvador, C., Contreras, M., and Escamilla, J.: On the Application of the Clark Electrode to the Study of Enzyme Kinetics in Apolar Solvents: The Catalase Reaction , Anal Biochem 184, 139, 1990
Evans, W., and Recheigl, M.: Factors Influencing Myeloperoxidase and Catalase Activities in Polymorphonuclear Leukocytes , Biochim Biophys Acta 148, 243, 1967
Feinstein, R., Braun, J., and Howard, J.: Acatalasemic and Hypocatalasemic Mouse Mutants. II. Mutational Variations in Blood and Solid Tissue Catalases , Arch Biochem Biophys 120, 165, 1967
Feinstein, R., Sacher, G., Howard, J., and Braun, J.: Comparative Heat Stability of Blood Catalase , Arch Biochem Biophys 122, 338, 1967
Fita, I., and Rossmann, M.: The NADPH Binding Site on Beef Liver Catalase , Proc Natl Acad Sci U S A 82,, 1985
Furuta, H., Hachimori, A., Ohta, Y., and Samejima, T.: Dissociation of Bovine Liver Catalase into Subunits on Acetylation , J Biochem (Tokyo) 76, 481, 1974
Ghadermarzi, M., and Moosavi-Movahedi, A.: Determination of the Kinetic Parameters for the 'Suicide Substrate' Inactivation of Bovine Liver Catalase by Hydrogen Peroxide , J Enzym Inhib 10, 167-175, 1996
Goldacre, P., and Galston, A.: The Specific Inhibition of Catalase by Substituted Phenols , Arch Biochem Biophys 43, 169, 1953
Goldstein, D.: A Method for Assay of Catalase with the Oxygen Cathode , Anal Biochem 24, 431, 1968
Goth, L.: Human Erythrocyte Catalase, Isolation with an Improved Method, Characterization and Comparison to Bovine Liver Catalase , Enzyme 41, 191, 1989
Goth, L.: A Simple Method for Determination of Serum Catalase Activity and Revision of Reference Range , Clin Chim Acta 196, 143, 1991
Gregory, E., and Fridovich, I.: Visualization of Catalase on Acrylamide Gels , Anal Biochem 58, 57, 1974
Haining, J., and Legan, J.: Improved Assay for Catalase Based Upon Steady-State Substrate Concentration , Anal Biochem 45, 469, 1972
Halliwell, B.: Superoxide Dismutase: A Contaminant of Bovine Catalase , Biochem J 135, 379, 1973
Heidrich, H.: New Aspects on the Heterogeneity of Beef Liver Catalase , Hoppe Seylers Z Physiol Chem 349, 873, 1968
Higashi, T., Kawamata, F., and Sakamoto, T.: Studies on Rat Liver Catalase. VII. Double-Labeling of Catalase by 14C-Leucine and 3H-d-Aminolevulinic Acid , J Biochem (Tokyo) 76, 703, 1974
Holmes, R.: Catalase Multiplicity in Normal and Acatalasemic Mice , FEBS Lett 24, 161, 1972
Holmes, R., and Masters, C.: Species Specific Features of the Distribution and Multiplicity of Mammalian Liver Catalase , Arch Biochem Biophys 148, 217, 1972
Johansson, L., and Borg, L.: A Spectophotometric Method for Determination of Catalase Activity in Small Tissue Samples , Anal Biochem 174, 331, 1988
Jones, G., and Masters, C.: On the Nature and Characteristics of the Multiple Forms of Catalase in Mouse Liver, Arch Biochem Biophys 169, 7, 1975
Jones, P.: The Evolution of Catalase Activity, Oxidases and Related Redox Systems,T. King, H. Mason, and M. Morrison, University Park Press, Baltimore, USA, 333, 1973
Jones, P., and Middlemiss, D.: Formation of Compound I by the Reaction of Catalase with Peroxoacetic Acid , Biochem J 130, 411, 1972
Kirkman, H., and Gaetani, G.: Catalase: A Tetrameric Enzyme with Four Tightly Bound Molecules of NADPH , Proc Natl Acad Sci U S A 81, 4243, 1984
Kiselev, N., Shpitzberg, C., and Vainshtein, B.: Crystallization of Catalase in the Form of Tubes with Monomolecular Walls , J Mol Biol 25, 433, 1967
Kremer, M.: Peroxidatic Activity of Catalase , Biochim Biophys Acta 198, 199, 1970
Kroll, R., Frears, E., and Bayliss, A.: An Oxygen Electrode-Based Assay of Ctalase Activity as a Rapid Method for Estimating the Bacterial Content of Foods, J Appl Bacteriol 66, 209, 1989
Kyle, M., Miccadei, S., Nakae, D., and Farber, J.: Superoxide Dismutase and Catalase Protect Cultured Hepatocytes from the Cytotoxicity of Acetaminophen , Biochem Biophys Res Commun 149, 889, 1987
Lanir, A., and Schejter, A.: On the Sixth Coordination Position of Beef Liver Catalase , FEBS Lett 55, 254, 1975
Lardinois, O.: Reactions of Bovine Liver Catalase with Superoxide Radicals and Hydrogen Peroxide , Free Radic Res 22, 251, 1995
Lee, K., Cronenwett, J., Shlafer, M., Corpron, C., and Zelenock, G.: Effect of Superoxide Dismutase Plus Catalase on Ca2+ Transport in Ischemic and Reperfused Skeletal Muscle , J Surg Res 42, 24, 1987
Lieberman, I., and Ove, P.: Catalase Requirement for Mammalian Cells in Culture , J Exp Med 108, 631, 1958
Loewen, P., and Seitala, J.: Purification and Characterization of Catalase-1 From Bacillus subtilis , Biochem Cell Biol 65, 939, 1987
Longley, W.: The Crystal Structure of Bovine Liver Catalase: A Combined Study by X-ray Diffraction and Electron Microscopy , J Mol Biol 30, 323, 1967
Maehly, A., and Chance, B.: The Assay of Catalases and Peroxidases, in, Methods of Biochemical Analysis,, , 357, 1954
Maimoni Goncalves, V., Cezar de Cerqueira Leite, L., Raw, I., and Cabrera-Crespo, J.: Purification of Catalase from Human Placenta , Biotechnol Appl Biochem 29, 73, 1999
Margoliash, E., and Novogrodsky, A.: A Study of the Inhibiton of Catalase by 3-Amino-1:2:4 Triazole , Biochem J 68, 468, 1958
Marklund, S.: Interactions Between Hydroxymethylhydroperoxide and Catalase , Biochim Biophys Acta 289, 269, 1972
Masters, C., Pegg, M., and Crane, D.: On the Multiplicity of the Enzyme Catalase in Mammalian Liver , Mol Cell Biochem 70, 113, 1986
McPherson, A., and Rich, A.: Crystallographic Study of Beef Liver Catalase , Arch Biochem Biophys 157, 23, 1973
Mitchel, R., and Anderson, I.: Catalase Photoinactivation, Science 150, 74, 1965
Miyahara, T., Takeda, A., Hachimori, A., and Samejima, T.: On the Heterogeneity of Catalase from Goat Liver, J Biochem (Tokyo) 84, 1267, 1978
Murphy, J., and Maier, R.: Intracellular Distribution of Iron, Catalase, and Protein in Tomato Plant Tissue, J Agric Food Chem 15, 113, 1967
Nadler, V., Goldberg, I., and Hochman, A.: Comparative Study of Bacterial Catalases, Biochim Biophys Acta 882, 234, 1986
Nelson, D., and Niesow, L.: Enthalphy of Decomposition of Hydrogen Peroxide by Catalase at 25deg.C (With Molar Extinction Coefficients of H2O2 Solution in the UV , Anal Biochem 49, 474, 1972
Nicholls, P., and Schonbaum, G.: Catalases, The Enzymes, 2nd Ed. Vol. 7,P. Boyer, H. Lardy, and K. Myrback, Academic Press, NY, 147, 1963
Orr, C.: Studies on Ascorbic Acid. I. Factors Influencing the Ascorbate-Mediated Inhibition of Catalase , Biochem 6, 2995, 1967
Orr, C.: Studies on Ascorbic Acid. II. Physical Changes in Catalase Following Incubation with Ascorbate and Copper, Biochem 6, 3000, 1967
Oshino, N., Jamieson, D., Sugano, T., and Chance, B.: Optical Measurements of the Catalase-Hydrogen Peroxide Intermediate (Compound) in the Liver of Anaesthetized Rats and Its Implication to Hydrogen Peroxide Production , Biochem J 146, 67, 1975
Oshino, N., Oshino, R., and Chance, B.: The Characteristics of the "Peroxidatic" Reaction of Catalase in Ethanol Oxidation , Biochem J 131, 555, 1973
Percy, M., Dalton, A., Markovic, V., McLachlan, D., Hummel, J., Rusk, A., and Andrews, D.: Red Cell Superoxide Dismutase, Glutathione Peroxidase and Catalase in Down Syndrome Patients with and without Manifestations of Alzheimer Disease , Amer J Med Genetics 35, 459, 1990
Perrin, R., Briancon, S., Jeandel, C., Artur, Y., Minn, A., Penin, F., and Siest, G.: Blood Activity of Cu/Zn Superoxide Dismutase, Glutathione Peroxidase and Catalase in Alzheimer's Disease - A Case-Control Study , Gerontology 36, 306, 1990
Pitts, J.E.: Crystallization by Centrifugation , Nature 355, 117, 1992
Poznansky, M., and Chang, T.: Comparison of the Enzyme Kinetics and Immunological Properties of Catalase Immobilized by Microencapsulation and Catalase in Free Solution for Enzyme Replacement, Biochim Biophys Acta 334, 103, 1974
Pritchard, G., and Hudson, M.: Changes in Catalase Activity in Higher Plants and Fungi treated with Oxygen at High Pressure, Nature 214, 945, 1967
Rorth, M., and Jensen, P.: Determination of Catalase Activity by Means of the Clark Oxygen Electrode, Biochim Biophys Acta 139, 171, 1967
Rossman, M., and Labaw, L.: A Comparison of Electron Microscopy and X-Ray Diffraction Results for Ox Liver Catalase Crystals, J Mol Biol 29, 315, 1967
Sakamoto, T., and Higashi, T.: Studies on Rat Liver Catalase. VIII. Isolation and Translation of Catalase Messenger RNA , J Biochem (Tokyo) 76, 1227, 1974
Samejima, T., and Kita, M.: The Conformational Changes of Catalase Molecule Caused by Ligand Molecules , Biochim Biophys Acta 175, 24, 1969
Samejima, T., Kamata, M., and Shibata, K.: Dissociation of Bovine Liver Catalase at Low pH , J Biochem (Tokyo) 51, 181, 1962
Schroeder, W., Shelton, J., Shelton, J., Robberson, B., and Apell, G.: The Amino Acid Sequence of Bovine Liver Catalase: A Preliminary Report , Arch Biochem Biophys 131, 652, 1969
Scibior, D., and Czeczot, H.: Catalase: Structure, Properties, Functions , Postepy Hig Med Dosw (Online) Vol. 60,, , 170, 2006
Scott, M., Lubin, B., Zuo, L., and Kuypers, F.: Erythrocyte Defense Against Hydrogen Peroxide - Preeminent Importance of Catalase , J Lab Clin Med 118, 7, 1991
Seah, T., and Kaplan, J.: Purification and Properties of the Catalase of Bakers' Yeast , J Biol Chem 248, 2889, 1973
Seah, T., Bhatti, A., and Kaplan, J.: Novel Catalytic Proteins of Bakers' Yeast. I. An Atypical Catalase , Can J Biochem 51, 1551, 1973
Sharma, K., Andersson, L., Loehr, T., Terner, J., and Goff, H.: Comparative Spectral Analysis of Mammalian, Fungal, and Bacterial Catalases. Resonance Raman Evidence for Iron-Tyrosinate Coordination , J Biol Chem 264, 12772, 1989
Shimizu, N., Kobayashi, K., and Hayashi, K.: The Reaction of Superoxide Radical with Catalase. Mechanism of the Inhibition of Catalase by Superoxide Radical , J Biol Chem 259, 4414, 1984
Spreti, N., Bartoletti, A., Di Profio, P., Germani, R., and Savelli, G.: Effects of lonic and Zwitterionic Surfactants on the Stabilization of Bovine Catalase , Biotechnol Prog 11, 107, 1995
Sumner, J., and Dounce, A.: Crystalline Catalase, J Biol Chem 121, 417, 1937
Sumner, J., Dounce, A., and Frampton, V.: Catalase III, J Biol Chem 136, 343, 1940
Sund, H., Weber, K., and Molbert, E.: Dissociation of Beef Liver Catalase in its Subunits. German , Eur J Biochem 1, 400, 1967
Tanaka, K., Takeda, T., and Miyajima, K.: Cryoprotective Effect of Saccharides on Denaturation of Catalase by Freeze-Drying, Chem Pharm Bull (Tokyo) 39, 1091, 1991
Tanford, C., and Lovrien, R.: Dissociation of Catalase into Subunits, J Am Chem Soc 84, 1892, 1962
Tauber, H., and Petit, E.: Convenient Methods for Preparing Crystalline Catalase from Cow Liver, J Biol Chem 195, 703, 1952
Tudhope, G.: Red Cell Catalase in Health and in Disease with Reference to the Enzyme Activity in Anaemia , Clin Sci 33, 165, 1967
Vuilaume, M., Lafont, R., Hubert, M., Jouve, H., Calvayrac, R., and Best-Belpomme, M.: A New Property of Catalase: The Concerted Synthesis of Nucleotide Triphosphates, Bioelectrochem Bioenergetics 19, 541, 1988
Wheeler, C., Salzman, J., Elsayed, N., Omaye, S., and Korte, D.: Automated Assays for Superoxide Dismutase, Catalase, Glutathione Peroxidase, and Glutathione Reductase Activity , Anal Biochem 184, 193, 1990
Whiteside, C., and Hassan, H.: Role of Oxyradicals in the Inactivation of Catalase by Ozone , Free Radic Biol Med 5, 305, 1988
Yasukochi, Y., Nakamura, M., and Minakami, S.: Effect of Cobalt on the Synthesis and Degradation of Hepatic Catalase in vivo , Biochem J 144, 455, 1974
Zidoni, E., and Kremer, M.: Kinetics and Mechanism of Catalase Action. Formation of the Intermediate Complex , Arch Biochem Biophys 161, 658, 1974

## Patentansprüche

1. Verfahren zur Ozonolyse von ungesättigten Fettsäuren und Derivaten davon umfassend die Schritte:
a) Ozonolyse einer ungesättigten Fettsäure oder eines Derivates davon in einer organischen Phase umfassend ein organisches Lösungsmittel, und
b) Kontaktieren der organischen Phase mit einer wässrigen Phase umfassend Katalase und bevorzugt einen Puffer,
wobei die ungesättigte Fettsäure oder das Derivat davon eine lineare Kette mit wenigstens acht Kohlenstoffatomen aufweist und
wobei es sich bei der ungesättigten Fettsäure oder dem Derivat davon um eine Verbindung der Formel (I)
R¹-(CH₂)ₓ-CH=CH-(CH₂)_{y}-COR² (I),
handelt,
wobei R¹ aus der Gruppe ausgewählt ist, die Wasserstoff, HO-, HS-, CHO-, R²OC-, unsubstituiertes oder subsitituiertes, bevorzugt halogeniertes, Alkyl, Cycloalkyl, Heterocycloalkyl, Aryl und Heteroaryl umfasst,
wobei R² aus der Gruppe ausgewählt ist, die HO-, substituiertes oder unsubstituiertes Alkoxy, Alkenoxy, Alkinoxy und Cycloalkoxy mit einem bis sieben Kohlenstoffatomen, -NR³R⁴, Aryloxy und Benzyloxy umfasst,
wobei R³ und R⁴ jeweils und unabhängig voneinander aus der Gruppe ausgewählt ist, die Wasserstoff, Alkyl, bevorzugt Methyl oder Ethyl, Alkenyl, Alkinyl, Aryl und Benzyl umfasst,
und wobei x und y jeweils und unabhängig voneinander 0 oder eine größere ganze Zahl sind.

2. Verfahren nach Anspruch 1, wobei das organische Lösungsmittel ein organisches Lösungsmittel ist, das in Anwesenheit einer wässrigen Phase bei Raumtemperatur eine von der wässrigen Phase separate organische Phase ausbildet.

3. Verfahren nach Anspruch 2, wobei Schritt a) in Anwesenheit einer wässrigen Phase erfolgt.

4. Verfahren nach Anspruch 3, wobei vor Schritt b) die wässrige Phase aus Schritt a) entfernt wird.

5. Verfahren nach einem der Ansprüche 1 bis 4, wobei R¹ Wasserstoff ist, R² HO- oder Methoxy oder Ethoxy ist und die Summe von x und y neun oder mehr beträgt.

6. Verfahren nach einem der Ansprüche 1 bis 5, wobei die Fettsäure bzw. das Derivat aus der Gruppe ausgewählt ist, die Ölsäure und Erucasäure und deren Ester, bevorzugt Methylester umfasst.

7. Verfahren nach einem der Ansprüche 1 bis 6, wobei das organische Lösungsmittel aus der Gruppe ausgewählt ist, die R⁵R⁶R⁷C-O-R⁸ oder intramolekulare Ether davon umfasst,
wobei R⁵, R⁶ und R⁷ jeweils und unabhängig voneinander aus der Gruppe ausgewählt sind, die Wasserstoff, ein Alkyl oder Cycloalkyl mit einem bis sieben Kohlenstoffatomen, sowie unsubstituiertes und substituiertes Aryl umfasst, wobei R⁸ aus der Gruppe ausgewählt ist, die Wasserstoff, ein Alkyl, Cycloalkyl, Hydroxyalkyl und Alkoxyalkyl mit einem bis sieben Kohlenstoffatomen sowie unsubstituiertes oder substituiertes Aryl und Benzyl umfasst, und wobei bevorzugt wenigstens einer der Reste R⁵, R⁶ und R⁷ Methyl ist.

8. Verfahren nach Anspruch 7, wobei es sich bei dem organischen Lösungsmittel um eine Verbindung der Formel R⁵R⁶R⁷C-OH handelt, und R⁵, R⁶ und R⁷ jeweils und unabhängig voneinander aus der Gruppe ausgewählt sind, die Wasserstoff, Methyl, Ethyl, Propyl, Butyl und Pentyl umfasst.

9. Verfahren nach Anspruch 8, wobei es sich bei dem organischen Lösungsmittel um 2-Methyl-2-butanol handelt.

10. Verfahren nach einem der Ansprüche 1 bis 9, wobei der Volumenanteil der wässrigen Phase beim Kontaktieren der organischen Phase am Gesamtvolumen beider Phasen mehr als 0 bis 50, bevorzugter 5 bis 30, noch bevorzugter 15 bis 25 Prozent beträgt.

11. Verfahren nach einem der Ansprüche 1 bis 10, wobei die Katalase bei Schritt a) anwesend ist.

12. Verfahren nach einem der Ansprüche 1 bis 11, wobei es sich bei der Katalase um Rinderleberkatalase oder eine Variante davon handelt.

13. Verfahren nach einem der Ansprüche 1 bis 12, wobei der pH Wert der wässrigen Phase in Schritt b) vor dem Kontaktieren mit der organischen Phase 3 bis 11, bevorzugt 5 bis 8, noch bevorzugter 6, 5 bis 7,5 beträgt.

14. Verwendung von Katalase zur Entfernung organischer reaktiver Sauerstoffspezies aus einem Reaktionsgemisch umfassend Ozonolyseprodukte einer ungesättigten Fettsäure oder eines Derivates, wie in Anspruch 1 definiert, davon.

15. Verwendung nach Anspruch 14, wobei es sich bei der ungesättigten Fettsäure oder dem Ester davon um eine Verbindung der Formel (I)
R¹-(CH₂)ₓ-CH=CH-(CH₂)_{y}-COR² (I),
handelt,
wobei R¹ aus der Gruppe ausgewählt ist, die Wasserstoff, HO-, HS-, CHO-, R²OC-, unsubstituiertes oder substituiertes, bevorzugt halogeniertes, Alkyl, Cycloalkyl, Heterocycloalkyl, Aryl und Heteroaryl umfasst,
wobei R² aus der Gruppe ausgewählt ist, die HO-, substituiertes oder unsubstituiertes Alkoxy, Alkenoxy, Alkinoxy und Cycloalkoxy mit einem bis sieben Kohlenstoffatomen, -NR³R⁴, Aryloxy und Benzyloxy umfasst,
wobei R³ und R⁴ jeweils und unabhängig voneinander aus der Gruppe ausgewählt ist, die Wasserstoff, Alkyl, bevorzugt Methyl oder Ethyl, Alkenyl, Alkinyl, Aryl und Benzyl umfasst,
und wobei x und y jeweils und unabhängig voneinander 0 oder eine größere ganze Zahl sind.

16. Verwendung nach einem der Ansprüche 14 bis 15, wobei das Reaktionsgemisch weiter ein organisches Lösungsmittel enthält, bevorzugt eines, das in Anwesenheit von einer wässrigen Phase bei Raumtemperatur eine von der wässrigen Phase separate organische Phase ausbildet
wobei das organische Lösungsmittel aus der Gruppe ausgewählt ist, die R⁵R⁶R⁷C-O-R⁸ oder intramolekulare Ether davon umfasst,
wobei R⁵, R⁶ und R⁷ jeweils und unabhängig voneinander aus der Gruppe ausgewählt sind, die Wasserstoff, ein Alkyl oder Cycloalkyl mit einem bis sieben Kohlenstoffatomen, sowie unsubstituiertes und substituiertes Aryl umfasst, wobei R⁸ aus der Gruppe ausgewählt ist, die Wasserstoff, ein Alkyl, Cycloalkyl, Hydroxyalkyl und ein entsprechendes Alkoxyalkyl mit einem bis sieben Kohlenstoffatomen sowie unsubstituiertes oder substituiertes Aryl und Benzyl umfasst, und wobei bevorzugt wenigstens einer der Reste R⁵, R⁶ und R⁷ Methyl ist,
und wobei es sich bei dem organischen Lösungsmittel noch bevorzugter um 2-Methyl-2-butanol handelt.

## Claims

1. Method for the ozonolysis of unsaturated fatty acids and derivatives thereof, comprising the steps:
a) ozonolysis of an unsaturated fatty acid or a derivative thereof in an organic phase comprising an organic solvent, and
b) contacting the organic phase with an aqueous phase comprising catalase and preferably a buffer,
where the unsaturated fatty acid or the derivative thereof has a linear chain with at least eight carbon atoms and
where the unsaturated fatty acid or the derivative thereof is a compound of the formula (I)
R¹-(CH₂)ₓ-CH=CH-(CH₂)_{y}-COR² (I),
where R¹ is selected from the group which comprises hydrogen, HO-, HS-, CHO-, R²OC-, unsubstituted or substituted, preferably halogenated, alkyl, cycloalkyl, heterocycloalkyl, aryl and heteroaryl,
where R² is selected from the group which comprises HO-, substituted or unsubstituted alkoxy, alkenoxy, alkynoxy and cycloalkoxy having one to seven carbon atoms, -NR³R⁴, aryloxy and benzyloxy,
where R³ and R⁴ in each case, and independently of one another, are selected from the group which comprises hydrogen, alkyl, preferably methyl or ethyl, alkenyl, alkynyl, aryl and benzyl,
and where x and y, in each case and independently of one another are 0 or a larger integer.

2. Method according to Claim 1, where the organic solvent is an organic solvent which, in the presence of an aqueous phase at room temperature forms an organic phase separate from the aqueous phase.

3. Method according to Claim 2, where step a) takes place in the presence of an aqueous phase.

4. Method according to Claim 3, where, before step b), the aqueous phase is removed from step a).

5. Method according to one of Claims 1 to 4, where R¹ is hydrogen, R² is HO- or methoxy or ethoxy and the sum of x and y is nine or more.

6. Method according to one of Claims 1 to 5, where the fatty acid or the derivative is selected from the group which comprises oleic acid and erucic acid and esters thereof, preferably methyl ester.

7. Method according to one of Claims 1 to 6, where the organic solvent is selected from the group which comprises R5R6R7C-O-R8 or intramolecular ethers thereof,
where R⁵, R⁶ and R⁷ in each case, and independently of one another, are selected from the group which comprises hydrogen, an alkyl or cycloalkyl having one to seven carbon atoms, and also unsubstituted and substituted aryl, where R⁸ is selected from the group which comprises hydrogen, an alkyl, cycloalkyl, hydroxyalkyl and alkoxyalkyl having one to seven carbon atoms, and unsubstituted or substituted aryl and benzyl, and where preferably at least one of the radicals R⁵, R⁶ and R⁷ is methyl.

8. Method according to Claim 7, where the organic solvent is a compound of the formula R⁵R⁶R⁷C-OH, and R⁵, R⁶ and R⁷ in each case, and independently of one another, are selected from the group which comprises hydrogen, methyl, ethyl, propyl, butyl and pentyl.

9. Method according to Claim 8, where the organic solvent is 2-methyl-2-butanol.

10. Method according to one of Claims 1 to 9, where the volume fraction of the aqueous phase upon contacting the organic phase, relative to the total volume of both phases, is more than 0 to 50, more preferably 5 to 30, even more preferably 15 to 25 percent.

11. Method according to one of Claims 1 to 10, where the catalase is present in step a).

12. Method according to one of Claims 1 to 11, where the catalase is beef liver catalase or a variant thereof.

13. Method according to one of Claims 1 to 12, where the pH of the aqueous phase in step b), before contacting with the organic phase, is 3 to 11, preferably 5 to 8, yet more preferably 6.5 to 7.5.

14. Use of catalase for removing organic reactive oxygen species from a reaction mixture comprising ozonolysis products of an unsaturated fatty acid or a derivative, as defined in Claim 1, thereof.

15. Use according to Claim 14, where the unsaturated fatty acid or the ester thereof is a compound of the formula (I)
R¹-(CH₂)ₓ-CH=CH-(CH₂)_{y}-COR² (I),
where R¹ is selected from the group which comprises hydrogen, HO-, HS-, CHO-, R²OC-, unsubstituted or substituted, preferably halogenated, alkyl, cycloalkyl, heterocycloalkyl, aryl and heteroaryl,
where R² is selected from the group which comprises HO-, substituted or unsubstituted alkoxy, alkenoxy, alkynoxy and cycloalkoxy having one to seven carbon atoms, -NR³R⁴, aryloxy and benzyloxy,
where R³ and R⁴ in each case, and independently of one another, are selected from the group which comprises hydrogen, alkyl, preferably methyl or ethyl, alkenyl, alkynyl, aryl and benzyl,
and where x and y, in each case and independently of one another are 0 or a larger integer.

16. Use according to one of Claims 14 to 15, where the reaction mixture further comprises an organic solvent, preferably one which, in the presence of an aqueous phase at room temperature, forms an organic phase separate from the aqueous phase,
where the organic solvent is selected from the group which comprises R⁵R⁶R⁷C-O-R⁸ or intramolecular ethers thereof,
where R⁵, R⁶ and R⁷ in each case, and independently of one another, are selected from the group which comprises hydrogen, an alkyl or cycloalkyl having one to seven carbon atoms, and also unsubstituted and substituted aryl, where R⁸ is selected from the group which comprises hydrogen, an alkyl, cycloalkyl, hydroxyalkyl and a corresponding alkoxyalkyl having one to seven carbon atoms, and unsubstituted or substituted aryl and benzyl, and where preferably at least one of the radicals R⁵, R⁶ and R⁷ is methyl,
and where the organic solvent is yet more preferably 2-methyl-2-butanol.

## Revendications

1. Procédé pour l'ozonolyse d'acides gras insaturés et de dérivés de ceux-ci, comprenant les étapes :
a) ozonolyse d'un acide gras insaturé ou d'un dérivé de celui-ci dans une phase organique comprenant un solvant organique et
b) mise en contact de la phase organique avec une phase aqueuse comprenant une catalase et de préférence un tampon,
l'acide gras insaturé ou le dérivé de celui-ci présentant une chaîne linéaire comprenant au moins huit atomes de carbone et
l'acide gras insaturé ou le dérivé de celui-ci étant un composé de formule (I)
R¹-(CH₂)ₓ-CH=CH-(CH₂)_{y}-COR² (I),
dans laquelle R¹ est choisi dans le groupe comprenant hydrogène, HO-, HS-, CHO-, R²OC- ; alkyle, cycloalkyle, hétérocycloalkyle, aryle et hétéroaryle, substitués ou non substitués, de préférence halogénés,
R² est choisi dans le groupe comprenant HO- ; alcoxy, alcénoxy, alcynoxy et cycloalcoxy substitués ou non substitués, comprenant un à sept atomes de carbone ;-NR³R⁴, aryloxy et benzyloxy,
R³ et R⁴ étant choisis, à chaque fois et indépendamment l'un de l'autre, dans le groupe comprenant hydrogène, alkyle, de préférence méthyle ou éthyle, alcényle, alcynyle, aryle et benzyle,
et x et y représentent, à chaque fois et indépendamment l'un de l'autre, 0 ou un nombre entier supérieur.

2. Procédé selon la revendication 1, le solvant organique étant un solvant organique qui, en présence d'une phase aqueuse à température ambiante, forme une phase organique séparée de la phase aqueuse.

3. Procédé selon la revendication 2, l'étape a) ayant lieu en présence d'une phase aqueuse.

4. Procédé selon la revendication 3, la phase aqueuse de l'étape a) étant séparée avant l'étape b).

5. Procédé selon l'une quelconque des revendications 1 à 4, R¹ représentant hydrogène, R² représentant HO- ou méthoxy ou éthoxy et la somme de x et y valant neuf ou plus.

6. Procédé selon l'une quelconque des revendications 1 à 5, l'acide gras ou le dérivé étant choisi dans le groupe qui comprend l'acide oléique et l'acide érucasique et leurs esters, de préférence l'ester méthylique.

7. Procédé selon l'une quelconque des revendications 1 à 6, le solvant organique étant choisi dans le groupe qui comprend R⁵R⁶R⁷C-O-R⁸ ou des éthers intramoléculaires correspondants,
R⁵, R⁶ et R⁷ étant choisis, à chaque fois et indépendamment les uns des autres, dans le groupe qui comprend hydrogène, alkyle ou cycloalkyle comprenant un à sept atomes de carbone, ainsi qu'aryle non substitué et substitué, R⁸ étant choisi dans le groupe qui comprend hydrogène, alkyle, cycloalkyle, hydroxyalkyle et alcoxyalkyle comprenant un à sept atomes de carbone ainsi qu'aryle et benzyle non substitué ou substitué, et de préférence un des radicaux R⁵, R⁶ et R⁷ représentant méthyle.

8. Procédé selon la revendication 7, où il s'agit, pour le solvant organique, d'un composé de formule R⁵R⁶R⁷C-OH et R⁵, R⁶ et R⁷ sont choisis, à chaque fois et indépendamment les uns des autres, dans le groupe comprenant hydrogène, méthyle, éthyle, propyle, butyle et pentyle.

9. Procédé selon la revendication 8, où il s'agit, pour le solvant organique, de 2-méthyl-2-butanol.

10. Procédé selon l'une quelconque des revendications 1 à 9, la proportion volumique de la phase aqueuse lors de la mise en contact avec la phase organique par rapport au volume total des deux phases étant supérieure à 0 à 50, plus préférablement de 5 à 30, encore plus préférablement de 15 à 25%.

11. Procédé selon l'une quelconque des revendications 1 à 10, la catalase étant présente lors de l'étape a).

12. Procédé selon l'une quelconque des revendications 1 à 11, où il s'agit, pour la catalase, d'une catalase de foie de boeuf ou d'un variant de celle-ci.

13. Procédé selon l'une quelconque des revendications 1 à 12, le pH de la phase aqueuse dans l'étape b), avant la mise en contact avec la phase organique, étant de 3 à 11, de préférence de 5 à 8, encore plus préférablement de 6,5 à 7,5.

14. Utilisation d'une catalase pour l'élimination d'espèces oxygénées réactives organiques d'un mélange réactionnel comprenant des produits d'ozonolyse d'un acide gras insaturé ou d'un dérivé de celui-ci, tel que défini dans la revendication 1.

15. Utilisation selon la revendication 14, l'acide gras insaturé ou l'ester de celui-ci étant un composé de formule (I)
R¹-(CH₂)ₓ-CH=CH-(CH₂)_{y}-COR² (I),
dans laquelle R¹ est choisi dans le groupe comprenant hydrogène, HO-, HS-, CHO-, R²OC- ; alkyle, cycloalkyle, hétérocycloalkyle, aryle et hétéroaryle, substitués ou non substitués, de préférence halogénés,
R² est choisi dans le groupe comprenant HO- ; alcoxy, alcénoxy, alcynoxy et cycloalcoxy substitués ou non substitués, comprenant un à sept atomes de carbone ;-NR³R⁴, aryloxy et benzyloxy,
R³ et R⁴ étant choisis, à chaque fois et indépendamment l'un de l'autre, dans le groupe comprenant hydrogène, alkyle, de préférence méthyle ou éthyle, alcényle, alcynyle, aryle et benzyle,
et x et y représentent, à chaque fois et indépendamment l'un de l'autre, 0 ou un nombre entier supérieur.

16. Utilisation selon l'une quelconque des revendications 14 à 15, le mélange réactionnel contenant en outre un solvant organique, de préférence un solvant qui, en présence d'une phase aqueuse à température ambiante, forme une phase organique séparée de la phase aqueuse,
le solvant organique étant choisi dans le groupe qui comprend R⁵R⁶R⁷C-O-R⁸ ou des éthers intramoléculaires correspondants,
R⁵, R⁶ et R⁷ étant choisis, à chaque fois et indépendamment les uns des autres, dans le groupe qui comprend hydrogène, alkyle ou cycloalkyle comprenant un à sept atomes de carbone, ainsi qu'aryle non substitué et substitué, R⁸ étant choisi dans le groupe qui comprend hydrogène, alkyle, cycloalkyle, hydroxyalkyle et alcoxyalkyle correspondant comprenant un à sept atomes de carbone ainsi qu'aryle et benzyle non substitué ou substitué, et de préférence un des radicaux R⁵, R⁶ et R⁷ représentant méthyle,
et où il s'agit, pour le solvant organique, encore plus préférablement, de 2-méthyl-2-butanol.
